(19) Europäisches Patentamt
European Patent Office
Office européen des brevets

(11) Veröffentlichungsnummer : **0 519 298 B1**

(12) # EUROPÄISCHE PATENTSCHRIFT

(45) Veröffentlichungstag der Patentschrift :
**13.09.95 Patentblatt 95/37**

(51) Int. Cl.[6] : **G03F 7/038,** C07D 251/24

(21) Anmeldenummer : **92109652.5**

(22) Anmeldetag : **09.06.92**

(54) **Strahlungsempfindliche Sulfonsäureester und deren Verwendung.**

(30) Priorität : **19.06.91 DE 4120174**

(43) Veröffentlichungstag der Anmeldung :
**23.12.92 Patentblatt 92/52**

(45) Bekanntmachung des Hinweises auf die Patenterteilung :
**13.09.95 Patentblatt 95/37**

(84) Benannte Vertragsstaaten :
**DE FR GB**

(56) Entgegenhaltungen :
**EP-A- 0 137 452**
**DE-A- 3 628 046**

(73) Patentinhaber : **HOECHST AKTIENGESELLSCHAFT**
**D-65926 Frankfurt am Main (DE)**

(72) Erfinder : **Pawlowski, Georg, Dr.**
**Fritz-Kalle-Strasse 34**
**W-6200 Wiesbaden (DE)**
Erfinder : **Röschert, Horst, Dr.**
**Am Pfingstborn 16**
**W-6531 Ober Hilbersheim (DE)**
Erfinder : **Spiess, Walter, Dr.**
**Rheingaustrasse 20**
**W-6110 Dieburg (DE)**
Erfinder : **Przybilla, Klaus-Jürgen, Dr.**
**Beethovenstrasse 19**
**W-6000 Frankfurt am Main 1 (DE)**

**EP 0 519 298 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Strahlungsempfindliche Sulfonsäureester und deren Verwendung

Die Erfindung betrifft Sulfonsäureester des 2,4-Bis-trichlormethyl-6-(mono- oder dihydroxyphenyl)-[1,3,5[triazins sowie ein negativ arbeitendes strahlungsempfindliches Gemisch mit

a) einer Verbindung, die unter Einwirkung von aktinischer Strahlung starke Säure bildet,

b) einer Verbindung mit mindestens zwei durch Säure vernetzbaren Gruppen und

c) einem in Wasser unlöslichen, in wäßrig-alkalischen Lösungen löslichen oder zumindest quellbaren polymeren Bindemittel.

Die Erfindung betrifft weiterhin ein damit hergestelltes strahlungsempfindliches Aufzeichnungsmaterial, das zur Herstellung von Photoresists, elektronischen Bauteilen, Druckplatten oder zum Formteilätzen geeignet ist.

Die stetige Verkleinerung der Strukturen, beispielsweise in der Chip-Herstellung bis in den Bereich von weniger als 1 μm, erfordert veränderte lithographische Techniken. Um solche feinen Strukturen abzubilden, verwendet man Strahlung mit einer kurzen Wellenlänge, wie energiereiches UV-Licht, Elektronen- und Röntgenstrahlen. Das strahlungsempfindliche Gemisch muß an die kurzwellige Strahlung angepaßt sein. Eine Zusammenstellung der an das strahlungsempfindliche Gemisch gestellten Forderungen ist in dem Aufsatz von C.G. Willson "Organic Resist Materials - Theory and Chemistry" [Introduction to Microlithography, Theory, Materials, and Processing, Herausgeber L.F. Thompson, C.G. Willson, M.J. Bowden, ACS Symp. Ser., 219, 87 (1983), American Chemical Society, Washington] angegeben. Es bestand daher ein verstärkter Bedarf an strahlungsempfindlichen Gemischen, die in den neueren Technologien, wie der Mid- oder Deep-UV-Lithographie, [Belichtung z. B. mit Excimer-Lasern bei Wellenlängen von 305 nm (XeF), 248 nm (KrF), 193 nm (ArF)], der Elektronen- oder der Röntgenstrahl-Lithographie, einsetzbar sind, vorzugsweise darüber hinaus in einem weiten Spektralbereich empfindlich sind und dementsprechend auch in der konventionellen UV-Lithographie verwendet werden können.

Negativ arbeitende strahlungsempfindliche Gemische, die Bisazide als Vernetzer und von Isopren abgeleitete Bindemittel enthalten, sind bekannt. Sie werden als strahlungsempfindliche Schichten bei der Herstellung von Druckplatten, gedruckten Schaltungen und integrierten Schaltkreisen verwendet. Ihr Einsatz in der Mikrolithographie ist jedoch durch verschiedene technische Nachteile begrenzt. So ist es schwierig, qualitativ hochwertige Schichten ohne Fehlstellen (pinholes) herzustellen. Der Wärmestand solcher Mischungen ist unzureichend, d. h. die Resistbilder werden bei der Verarbeitung durch thermischen Fluß verzerrt. Schließlich ist ihr Auflösungsvermögen auf Strukturen > 2 μm begrenzt, da sie bei der notwendigen Entwicklung mit organischen Lösemitteln auch in den gehärteten Bereichen unerwünscht hohe Quellung zeigen, was wiederum zu Strukturverzerrungen oder inhomogenen Entwicklungsprozessen und damit zu ungenauer Wiedergabe des durch die Belichtungsmaske vorgegebenen Bildes führt. Um Resistbilder mit einer Auflösung von besser als 2 μm herstellen zu können, sind andere negativ arbeitende strahlungsempfindliche Gemische entwickelt worden, die empfindlich gegenüber Strahlung kürzerer Wellenlänge sind. Ein derartiges Gemisch enthält beispielsweise ein Copolymer aus (2,3-Epoxy-propyl)-methacrylat und (2,3-Di-chlor-propyl)methacrylat (DCOPA) oder eine Kombination der entsprechenden Homopolymeren. Die Glasübergangstemperatur dieses Gemisches ist jedoch für viele Anwendungen zu niedrig, insbesondere ist aber die geringe Plasmaätzbeständigkeit des Gemisches zu bemängeln. Darüber hinaus muß auch dieses Resistmaterial mit Entwicklern auf der Basis von wenig umweltfreundlichen, organischen Lösemitteln verarbeitet werden. Eine geringe Plasmaätzbeständigkeit zeigen auch andere bisher bekannte negativ arbeitende Photoresists auf aliphatischer Basis.

In der EP-A 0 164 248 wurde ein säurehärtbares Gemisch beschrieben, das wäßrig-alkalisch entwickelbar ist, durch die Verwendung von Aromaten eine verbesserte Plasmaätzresistenz aufweist und gegenüber nahem UV-Licht (350 bis 450 nm) empfindlich ist. Als Säurebildner sind dabei insbesondere Sulfonsäureesterderivate des Diazonaphthochinons genannt worden, die bei der Belichtung schwach saure Carbonsäuren bilden und daher nur in vergleichsweise hoher Konzentration wirksam sind. Solche Gemische haben aber infolge der schwachen Absorptionen und des unzureichenden Ausbleichverhaltens des photolytischen Säurebildners eine geringe Empfindlichkeit für Deep-UV-, Elektronen- und Röntgenstrahlung.

In der US-A 3,692,560 wird ein säurehärtbares Gemisch beschrieben, das ein säurevernetzbares Melaminderivat, einen Novolak und chlorierte Benzophenone als photolytische Säurebildner enthält. Auch diese Gemische haben im tiefen UV-Bereich keine ausreichende Empfindlichkeit.

Das gleiche trifft für die in der EP-A 0 232 972 erwähnten säurebildenden Derivate des DDT's zu, die hochtoxisch sind und schon von daher nicht praxisgerecht sein können. Immerhin zeigen solche Verbindungen eine beachtliche Empfindlichkeit im tiefen UV-Bereich (200 bis 300 nm).

Als Verbindungen, die bei Bestrahlung eine starke Säure bilden, wurden bisher insbesondere Onium-Salze, wie Diazonium-, Phosphonium-, Sulfonium- und Jodonium-Salze von nicht nucleophilen Säuren, wie $HSbF_6$, $HAsF_6$, oder $HPF_6$ [J.V. Crivello, Polym. Eng. Sci., 23 (1983) 953] verwendet. Daneben sind Halogenverbindungen, insbesondere Trichlormethyltriazin-Derivate (EP-A 0 137 452 = US-A 4,619,998/4,696,888) oder Trichlormethyloxadiazol-Derivate, o-Chinondiazidsulfonylchloride, o-Chinondiazid-4-sulfonsäureester, Bis(sulfonyl)diazomethane, Sulfonylcarbonyl-diazomethane oder Nitrobenzyltosylate empfohlen worden.

Diese Verbindungen werden in negativ oder positiv arbeitenden strahlungsempfindlichen Gemischen verwendet. Die Verwendung derartiger photolytischer Säurebildner bringt aber gewisse Nachteile mit sich, die ihre Einsatzmöglichkeiten in verschiedenen Anwendungsbereichen drastisch einschränken. So weisen die Oniumsalze eine relativ hohe, kaum bleichbare Eigenabsorption im gewünschten Wellenlängenbereich auf, was dazu führt, daß die maximal erreichbare Auflösung der resultierenden strahlungsempfindlichen Gemische in vielen Fällen limitiert ist. Ferner sind z.B. viele der Oniumsalze toxisch. Ihre Löslichkeit ist in vielen Lösemitteln unzureichend, weshalb nur wenige Lösemittel zur Herstellung einer Beschichtungslösung geeignet sind. Darüber hinaus werden bei Verwendung der Oniumsalze z.T. unerwünschte Fremdatome eingeführt, die insbesondere in der Mikrolithographie zu Prozeßstörungen führen können, so daß der Einsatz solcher Gemische zu unbefriedigenden Ergebnissen führt. Halogenverbindungen, wie z. B. Chinondiazidsulfonsäurechloride bilden zwar starke Halogenwasserstoffsäuren, besitzen jedoch im strahlungsempfindlichen Gemisch und auch auf bestimmten Substraten nur eine begrenzte Haltbarkeit, die dadurch verbessert wurde, daß zwischen Substrat und strahlungsempfindlicher, Verbindungen des Typs a) enthaltender Schicht eine Zwischenschicht eingefügt wurde, was allerdings zu einer unerwünschten Zunahme von Defekten und zu einer verminderten Reproduzierbarkeit führte (DE-A 36 21 376 = US-A 4,840,867).

Andere Halogenwasserstoffsäure generierende Verbindungsklassen, wie z. B. Bis(trichlormethyl)triazin-Derivate (EP-A 0 137 452), weisen zwar eine gute Säurebildungseffizienz auf, besitzen jedoch eine oft unzureichende Löslichkeit in gängigen Resistlösemitteln und werden dazu häufig unter den in der Praxis angewandten Verarbeitungsbedingungen aus der lichtempfindlichen Schicht ausgeschwitzt, so daß eine reproduzierbare Herstellung der resultierenden Schicht nicht mehr möglich ist.

In neueren Arbeiten von F.M. Houlihan et al., SPIE 920, 67 (1988) wurde anhand positiv arbeitender Systeme gezeigt, daß neben den oben genannten Säurebildnern auch Nitrobenzyltosylate, die bei Belichtung Sulfonsäuren mit geringer Wanderungstendenz bilden, in bestimmten säurelabilen Resistformulierungen verwendbar sind. Es kann aus diesen Ergebnissen abgeleitet werden, daß solche Verbindungen auch für photohärtbare Systeme verwendet werden können. Die dabei erzielten Empfindlichkeiten und die thermische Stabilität der Photoresists erwiesen sich jedoch als unzureichend.

Trotz der bisher geleisteten intensiven Forschungstätigkeit auf diesem Gebiet ist derzeit kein strahlungsempfindliches Gemisch bekannt, mit dem sich ein negativ arbeitendes strahlungsempfindliches Aufzeichnungsmaterial herstellen läßt, das eine hohe Empfindlichkeit im DUV-Bereich (200 bis 300 nm) sowie eine hohe Auflösung besitzt, mit den in der Praxis üblichen lithographischen Verarbeitungsschritten voll kompatibel und wäßrig-alkalisch entwickelbar ist.

Aufgabe der Erfindung war es daher, neue Verbindungen, die unter Einwirkung von aktinischer Strahlung starke Säure bilden, sowie ein damit hergestelltes strahlungsempfindliches Gemisch auf der Basis von säurebildenden in Kombination mit säurevernetzbaren Verbindungen zur Verfügung zu stellen, wobei die photolytisch eine Säure bildende Verbindung die zahlreichen oben näher bezeichneten Nachteile nicht aufweisen soll.

Die Aufgabe wird gelöst durch strahlungsempfindliche, mit Sulfonsäure(n) der Formel $R\text{-}SO_3H$ bzw. $R'(\text{-}SO_3H)_2$ veresterte 2,4-Bis-trichlormethyl-6-(mono- oder dihydroxyphenyl)-[1,3,5]triazine der allgemeinen Formeln I und/oder II

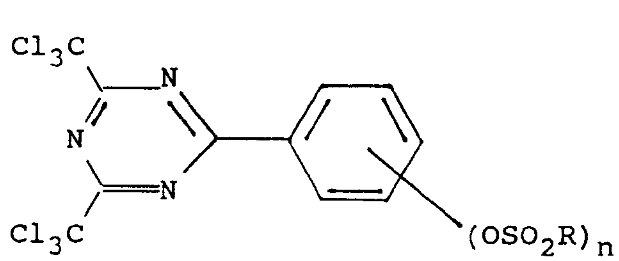

(I)

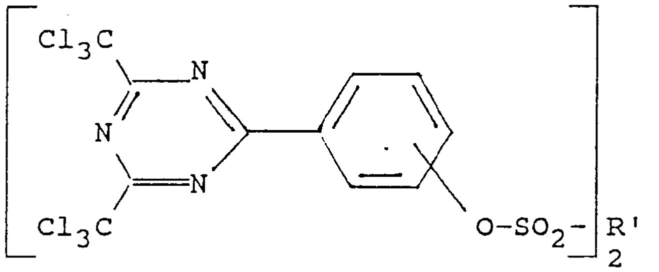

(II)

wobei R einen ggf. weiter substituierten ($C_1$-$C_{10}$)Alkyl-, ($C_5$-$C_{10}$)Cycloalkyl-, ($C_6$-$C_{10}$)Aryl-, ($C_6$-$C_{10}$)Aryl-($C_1$-$C_{10}$)alkyl-, ($C_3$-$C_9$)Heteroarylrest, 1-Anthracenyl oder 2-Anthrachinonyl, R′ einen ggf. substituierten ($C_1$-$C_{10}$)Alkylen-, ($C_6$-$C_{10}$)Arylen- oder ($C_3$-$C_9$)Heteroarylenrest bezeichnet und n 1 oder 2 sein kann.

Im Falle n = 2 können die Reste R auch verschieden sein.

Die Reste R und R′ können mit mindestens einem Substituenten ausgewählt aus der Gruppe bestehend aus ($C_1$-$C_8$)Alkyl, ($C_1$-$C_8$)Alkoxy, ($C_1$-$C_8$)Alkanoyl, ($C_1$-$C_8$)Alkanoyloxy, ($C_6$-$C_{10}$)Aryl, Cyano oder Halogen substituiert sein.

Die Reste R und R′ können, wie die folgenden Beispiele zeigen, gegebenenfalls auch noch andere Substituenten aufweisen:

Methyl, Ethyl, Propyl, Isopropyl, Butyl, Hexyl, Octyl, Decyl, Dodecyl, Hexadecyl, Octadecyl, 10-Camphyl, Chlormethyl, 2-Chlorethyl, 3-Chlorpropyl, Dichlormethyl, Trichlormethyl, Difluormethyl, Trifluormethyl, 2,2,2-Trifluorethyl, 1,1,2,3,3,3-Hexafluorpropyl, Perfluorhexyl, Trimethylsilylmethyl, Methansulfonylmethyl, Phenyl, Benzyl, 4-Acetylphenyl, 4-Acetylaminophenyl, 2-, 3- oder 4-Bromphenyl, 2-, 3- oder 4-Chlorphenyl, 2-, 3- oder 4-Fluorphenyl, 4-Jodphenyl, 2-, 3- oder 4-Methylphenyl, 4-Ethylphenyl, 4-Propylphenyl, 4-Isopropylphenyl, 4-Iso-4-tert.-Butylphenyl, 4-tert.-Amylphenyl, 4-Hexylphenyl, 4-Methoxyphenyl, 4-Butoxyphenyl, 4-Hexadecyloxyphenyl, 2-, 3- oder 4-Trifluormethylphenyl, 2- oder 4-Trifluormethoxyphenyl, 2-, 3- oder 4-Nitrophenyl, 3- oder 4-Carboxyphenyl, 2-Methoxycarbonylphenyl, 4-Tetrafluorethoxyphenyl, β-Styryl, 4-Acetylamino-3-chlorphenyl, 4-Acetylamino-3-fluorphenyl, 3,5-Bistrifluormethylphenyl, 2,5-Bis-(2,2,2-trifluorethoxy)phenyl, 2,5-Dimethylphenyl, 2,4-, 2,5- oder 3,4-Dimethoxyphenyl, 2,4-Diisopropylphenyl, 5-Brom-2-methoxyphenyl, 2- oder 3-Chlor-4-fluorphenyl, 3-Chlor-2-methylphenyl, 3-Chlor-4-methoxyphenyl, 2-Chlor-6-methylphenyl, 2-Chlor-4-trifluormethylphenyl, 5-Chlor-2-methoxyphenyl, 5-Fluor-2-methylphenyl, 2,5- oder 3,4-Dibromphenyl, 2,3-, 2,4- oder 2,5-, 2,6-, 3,4- oder 3,5-Dichlorphenyl, 2-(2,4-Dichlorphenoxy)phenyl, 4-(2-Chlor-6-nitrophenoxy)phenyl, 2,4- oder 2,5-Difluorphenyl, 3-Carboxy-4-chlorphenyl, 4-Chlor-3-nitrophenyl, 2-Methyl-5-nitrophenyl, 4-Chlor-3- oder 2-Chlor-5-trifluormethylphenyl, 4-(2,2-Dichlorcyclopropyl)phenyl, 2,4-Dinitrophenyl, 4-Dimethylamino-3-nitrophenyl, 2-Nitro-4-trifluormethylphenyl, 2,4,6-Trimethylphenyl, 2,4,6-Triisopropylphenyl, 2,3,4-, 2,4,5- oder 2,4,6-Trichlorphenyl, 4-Chlor-2,5-dimethylphenyl, 2,4-Dichlor-5-methylphenyl, 3,5-Dichlor-2-hydroxyphenyl, 3,5-Dichlor-4-(4-nitrophenoxy)phenyl, 4-(2-Chlor-4-nitrophenoxy)-3,5-Dichlorphenyl, 4-Brom-2,5-difluorphenyl, 2,4-Dimethyl-3-nitrophenyl, 3,5-Dinitro-4-methylphenyl, 2,3,5,6-Tetramethylphenyl, 4-Methoxy-2,3,6-trimethylphenyl, 2,5-Dibrom-3,6-difluorphenyl, 2,3,4,5,6-Pentafluorphenyl, 1- oder 2-Naphthyl, 5-Diazo-6-oxo-5,6-dihydro-1-naphthyl, 6-Diazo-5-oxo-5,6-dihydro-1-naphthyl, 5-Diazo-6-oxo-5,6-dihydro-8-naphthyl, 5-Diazo-3-methoxy-6-oxo-5,6-dihydro-8-naphthyl, 5-Dimethylamino-1-naphthyl, 1-Anthracenyl, 2-Anthrachinonyl, 8-Chinolinyl, 2-Thienyl, 5-Chlor-2-thienyl, 4-Brom-2,5-dichlor-3-thienyl, 4,5-Dibrom-2-thienyl, 2,3-Dichlor-5-thienyl, 2-Brom-3-Chlor-5-thienyl, 3-Brom-2-chlor-5-thienyl, 3-Brom-5-chlor-2-thienyl, 2,5-Dichlor-3-thienyl, 2-(2-Pyridyl)-5-thienyl, 5-Chlor-1,3-dimethyl-4-pyrazolyl, 3,5-Dimethyl-4-isoxazolyl, 2,4-Dimethyl-5-thiazolyl, 2-Acetylamino-4-methyl-5-thiazolyl

oder

1,4-Butylen, 2-Oxo-1,3-propylen, 1,2- oder 1,3-Phenylen, 3-Methyl-1,2-phenylen, 2,4,6-Trimethyl-1,3-phenylen, 4,4′-Biphenylen, 4,4′-Methylendiphenylen, 4,4′-Oxybiphenylen, 1,5-Naphthylen, 2-Chlor-3,5-thienylen, 2-(1-Methyl-5-trifluormethylpyrazol-3-yl)-3,5-thienylen.

Hinsichtlich der Stellung der Hydroxygruppe(n) am aromatischen Benzolring bestehen keinerlei Einschränkungen, d. h. die entsprechenden Cyanophenol-Vorstufen sind bezüglich des Substitutionsmusters frei wählbar.

Von den Verbindungen der allgemeinen Formel I sind solche mit n = 1 bevorzugt.

Das erfindungsgemäße strahlungsempfindliches Gemischs enthält

a) eine Verbindung, die unter Einwirkung von aktinischer Strahlung starke Säure bildet,

b) eine Verbindung mit mindestens zwei durch Säure vernetzbaren Gruppen und

c) ein in Wasser unlösliches, in wäßrig-alkalischen Lösungen lösliches oder zumindest quellbares polymeres Bindemittel,
dadurch gekennzeichnet, daß die Verbindung a) ein mit Sulfonsäure(n) der Formel $R-SO_3H$ bzw. $R'(-SO_3H)_2$ verestertes 2,4-Bis-trichlormethyl-6-(mono- oder dihydroxyphenyl)-[1,3,5]triazin mit der obengenannten Konstitution ist.

In speziellen strahlungsempfindlichen Gemischen können zur Verbesserung der Löslichkeit in gängigen Resistlösemitteln und/oder zur Optimierung des Löslichkeitsratenverhältnisses nicht vollständig veresterte Produkte, d. h. solche, die noch freie phenolische Hydroxygruppen aufweisen, bevorzugt eingesetzt werden.

Die Herstellung der erfindungsgemäßen, mit Sulfonsäuren der Formel $R-SO_3H$ bzw. $R'(-SO_3H)_2$ veresterten 2,4-Bistrichlormethyl-6-(mono- oder dihydroxyphenyl)-[1,3,5]-triazine ist an sich bekannt. Als Ausgangsmaterialien dienen dabei in erster Linie Cyanophenole und die entsprechenden Sulfonsäurechloride. Verfahren zu Herstellung von aromatischen Sulfonsäureestern werden beispielsweise von F. Muth in: Houben-Weyl-Müller, Methoden der organischen Chemie, Bd. IX, S. 633 (und dort zitierte Literatur), Thieme-Verlag, 4. Aufl., Stuttgart 1955, und von S. Pawlenko, a.a.O., Bd. E 11, S. 1084, Thieme-Verlag, 1. Aufl., Stuttgart 1985, sowie in der Patentliteratur an zahlreichen Beispielen beschrieben. Auch die entsprechenden Sulfonsäureanhydride sind als Ausgangsmaterialien geeignet (siehe S. Pawlenko, a.a.O., Bd. E11, S. 1086, Thieme-Verlag, 1. Aufl., Stuttgart 1985, und P. J. Stang, M. Hanack, L. R. Subramaniam, Synthesis, **1982**, 85). Dies gilt insbesondere für die mit Perfluoralkyl-Gruppen substituierten Benzolsulfonsäureanhydride. Die Herstellung der mit den Sulfonsäuren der Formel $R-SO_3H$ bzw. $R'(-SO_3H)_2$ veresterten 2,4-Bis-trichlormethyl-6-(mono- oder dihydroxyphenyl)-[1,3,5]triazine aus den entsprechenden mit Sulfonsäuren $R-SO_3H$ veresterten Cyanophenolen wurde analog zur EP-A 0 137 452 durchgeführt.

Das erfindungsgemäße strahlungsempfindliche Gemisch zeichnet sich durch eine hohe Empfindlichkeit über einen weiten Spektralbereich aus. Während der Verarbeitung unter praxisgerechten Bedingungen wurden keinerlei Einschränkungen bezüglich der Löslichkeit in gängigen Resistlösemitteln und bezüglich des Ausdampfens der strahlungsempfindlichen Komponente a) aus der Schicht beobachtet. Das strahlungsempfindliche Gemisch zeigt eine hohe thermische Stabilität und schafft die Möglichkeit, auch feinste Strukturen einer Vorlage detailgenau wiederzugeben.

Überraschenderweise ist die Quantenausbeute bei der Bestrahlung der erfindungsgemäßen Gemische größer als bei der Bestrahlung bekannter Gemische mit 2,4-Bis-trichlormethyl-[1,3,5]triazinen. Diese höhere Effizienz der Säurebildung deutet darauf hin, daß nicht nur Halogenwasserstoffsäuren, sondern auch - jedoch in geringerem Umfang - Sulfonsäuren bei der Bestrahlung generiert werden und im erfindungsgemäßen Gemisch zu einer gesteigerten Empfindlichkeit beitragen. Die Eignung der erfindungsgemäßen Säurebildner der allgemeinen Formel I in negativ arbeitenden Gemischen war deshalb nicht naheliegend, da bei der Bestrahlung u. a. auch gut lösliche Spaltprodukte (Phenole und Sulfonsäuren) in den zu vernetzenden Bereichen gebildet werden.

Die erfindungsgemäßen, negativ arbeitenden, strahlungsempfindlichen Gemische zeigen zudem nicht nur eine hohe thermische und Plasma-Beständigkeit sondern auch hervorragende lithographische Eigenschaften, die eine Auflösung im Halbmikrometer- und teilweise auch im Sub-Halbmikrometer-Bereich erlauben. Man erhält nach dem bildmäßigen Bestrahlen und anschließendem Entwickeln ein detailgetreues Abbild der Maske. Die Resistfelder weisen steile Flanken auf. In den nichtbestrahlten Bereichen wird die Resistschicht vollständig abgelöst, d. h. es verbleiben keinerlei Reste oder Rückstände der Schicht auf dem Substrat. Die bei der Photolyse gebildeten Säuren führen zu einer effizienten Vernetzung der Resist-Komponenten b) und c), was die Herstellung von hochempfindlichen, negativ arbeitenden Gemischen erlaubt.

In dem erfindungsgemäßen strahlungsempfindlichen Gemisch kann das bei Bestrahlung Säure bildende, mit Sulfonsäure(n) der Formel $R-SO_3H$ bzw. $R'(-SO_3H)_2$ veresterte 2,4-Bis-trichlormethyl-6-(mono- oder dihydroxyphenyl)-[1,3,5]triazin allein oder in Kombination mit anderen Säurebildnern Verwendung finden. Als solche wären z. B. andere Halogenverbindungen zu nennen, insbesondere Trichlormethyltriazinderivate oder Trichlormethyloxadiazolderivate, Onium-Salze, 1,2-Disulfone, o-Chinondiazidsulfonylchloride oder Organometall-Organohalogen-Kombinationen. Es kommen aber auch Mischungen mit Bis(sulfonyl)diazomethanen und Sulfonyl-carbonyldiazomethanen in Frage. In solchen Gemischen können jedoch die eingangs erwähnten Nachteile wieder auftreten.

Mit den erfindungsgemäßen Gemischen hergestellte Aufzeichnungsmaterialien zeigen überraschend eine höchsten Ansprüchen genügende Bilddifferenzierung und, noch überraschender, eine Verbesserung des Kontrasts und des Auflösungsvermögens. Die erfindungsgemäßen Gemische erlauben beispielsweise die Herstellung eines hochempfindlichen negativ arbeitenden Photoresists für energiereiche UV2-Strahlung (z. B. 248 nm).

Da das erfindungsgemäße Gemisch über einen weiten Spektralbereich empfindlich ist, ist zum bildmäßigen Bestrahlen aktinische Strahlung allgemein geeignet. Als aktinische Strahlung soll in diesem Zusammenhang jede Strahlung verstanden werden, deren Energie mindestens der des kurzwelligen sichtbaren Lichts

entspricht. Geeignet ist dabei insbesondere UV-Strahlung im Bereich von 190 bis 450 nm, bevorzugt von 200 bis 400 nm, besonders bevorzugt von 200 bis 300 nm, aber auch Elektronen- oder Röntgenstrahlung.

Der Anteil an mit Sulfonsäuren der allgemeinen Formel $R-SO_3H$ bzw. $R'(-SO_3H)_2$ veresterten 2,4-Bis-trichlormethyl-6-(mono- oder dihydroxyphenyl)-[1,3,5]triazinen im erfindungsgemäßen Gemisch liegt im allgemeinen bei 0,25 bis 15 Gew.-%, bevorzugt bei 0,5 bis 5 Gew.-%, bezogen auf das Gesamtgewicht der Feststoffe im Gemisch.

Als säurevernetzbare Verbindungen b) kommen besonders die in der GB 2,082,339 offenbarten Resole, daneben auch mit Alkoxymethyl- oder Oxiranylmethyl-Gruppen substituierte Aromaten (EP-A 0 212 482) sowie monomere und oligomere Melamin- bzw. Harnstoff-Formaldehyd-Kondensate (EP-A 0 133 216, DE-A 36 34 371, DE-A 37 11 264) in Betracht. Beispiele für den ersten Typ sind insbesondere die kommerziell erhältlichen Resolprodukte (R)Bakelite R 5363, (R)Bakelite R 17620, (R)Bakelite R 10282 und (R)Kelrez 40-152. Resolderivate sind jedoch insgesamt nicht bevorzugt, da sie im tiefen UV-Bereich verhältnismäßig hohe Absorptionen aufweisen und damit zu einer Beeinträchtigung der Bildwiedergabe führen.

Besser geeignet sind die aus der EP-A 0 212 482 bekannten Vernetzer der allgemeinen Formel **III**

$$(R^1O\text{-}CHR^3)_1\text{-}A\text{-}(CHR^3\text{-}OR^2)_m \qquad (III)$$

in der

| | |
|---|---|
| A | -B- oder -B-Y-B- ist und |
| B | einen ggf. substituierten einkernigen aromatischen Kohlenwasserstoff oder eine Sauerstoff oder Schwefel enthaltende heterocyclische aromatische Verbindung darstellt, |
| Y | eine Einfachbindung, $(C_1\text{-}C_4)$Alkylen oder $(C_1\text{-}C_4)$Alkylendioxy, deren Ketten durch Sauerstoffatome unterbrochen sein können, -O-, -S-, $-SO_2-$, -CO-, $-CO_2-$, $-O-CO_2-$, -CONH- oder $-O-C_6H_4-O-$ bedeutet, |
| $R^1$ und $R^2$ | Wasserstoff, $(C_1\text{-}C_6)$Alkyl, $C_5$ oder $C_6$-Cyclo-alkyl-, ggf. substituiertes $(C_6\text{-}C_{12})$Aryl, $(C_6\text{-}C_{12})$Aralkyl oder Acyl sind, |
| $R^3$ | Wasserstoff, $(C_1\text{-}C_4)$Alkyl oder ggf. substituiertes Phenyl, |
| l | eine ganze Zahl von 1 bis 3 und |
| m | eine ganze Zahl von 0 bis 3, wobei l+m mindestens 2 ist, bedeuten. |

Typische Vernetzer sind demnach Aromaten und Heterocyclen, die mehrfach mit Hydroxymethyl-, Acetoxymethylund Methoxymethyl-Gruppen substituiert sind.

Weitere bevorzugte Vernetzer sind Melamin/Formaldehyd-Derivate, die beispielsweise mindestens zwei freie N-Hydroxymethyl-, N-Alkoxymethyl- oder N-Acyloxymethylgruppen aufweisen. Insbesondere die N-Alkoxymethylderivate sind zum Einsatz in dem erfindungsgemäßen strahlungsempfindlichen Gemisch geeignet.

Die Vernetzer sind in der Lage, bei erhöhten Temperaturen unter dem Einfluß der photolytisch erzeugten Säuren mit den polymeren Bindemitteln zu vernetzen. Allgemein sind sie dadurch gekennzeichnet, daß sie unter den genannten Temperatur- und Säurebedingungen ein Carbonium-ion bilden können.

Der Anteil der säurevernetzbaren Verbindung b) am erfindungsgemäßen strahlungsempfindlichen Gemisch liegt zweckmäßig bei 1 bis 50 Gew.-%, vorzugsweise bei 5 bis 25 Gew.-%, jeweils bezogen auf das Gesamtgewicht der festen Bestandteile des Gemisches.

Das erfindungsgemäße strahlungsempfindliche Gemisch enthält ferner mindestens ein polymeres, in Wasser unlösliches, in wäßrig-alkalischen Lösungen dagegen lösliches, zumindest quellbares Bindemittel c). Das Bindemittel zeichnet sich im besonderen dadurch aus, daß es mit den übrigen Bestandteilen des erfindungsgemäßen strahlungsempfindlichen Gemisches gut verträglich ist und insbesondere im Wellenlängenbereich von 190 bis 300 nm eine möglichst geringe Eigenabsorption, d.h. eine hohe Transparenz, aufweist. Bindemittel auf der Basis von Novolak-Kondensationsharzen, die in der Regel in Kombination mit Naphthochinondiaziden als photoaktive Komponenten eingesetzt werden, erfüllen diese Bedingung nicht. Zwar lassen Novolak-Kondensationsharze nach bildmäßiger Belichtung in den nicht belichteten Bereichen eine Erniedrigung der Löslichkeit gegenüber wäßrig-alkalischen Entwicklern erkennen, doch ist ihre Eigenabsorption im Bereich der für die Bestrahlung gewünschten kurzen Wellenlänge unerwünscht hoch.

Novolak-Kondensationsharze können aber in Mischung mit anderen als Bindemittel geeigneten Harzen mit höherer Transparenz eingesetzt werden. Die Mischungsverhältnisse richten sich dabei vorwiegend nach der Art des mit dem Novolakharz zu mischenden Bindemittels. Insbesondere spielen dessen Grad an Eigenabsorption im genannten Wellenlängenbereich, aber auch die Mischbarkeit mit den anderen Bestandteilen des strahlungsempfindlichen Gemisches eine entscheidende Rolle. Im allgemeinen kann aber das Bindemittel des erfindungsgemäßen strahlungsempfindlichen Gemisches bis zu 30 Gew.-%, insbesondere bis zu 20 Gew.-%, eines Novolak-Kondensationsharzes enthalten.

Das Bindemittel(gemisch) weist vorteilhaft eine Extinktion für Strahlung der Wellenlänge 248 nm von $< 0{,}5\ \mu m^{-1}$, bevorzugt $< 0{,}3\ \mu m^{-1}$, auf.

Als Bindemittel geeignet sind Homo- oder Copolymere des p-Hydroxystyrols sowie seiner Alkylderivate,

z. B. des 3-Methyl-4-hydroxystyrols, Homo- oder Copolymere anderer Vinylphenole, z. B. des 3-Hydroxystyrols, und der Ester oder Amide von Acrylsäure mit phenolischen Gruppen aufweisenden Aromaten. Als Comonomere können polymerisierbare Verbindungen wie Styrol, Methyl(meth)acrylat oder ähnliche eingesetzt werden.

Gemische mit erhöhter Plasmabeständigkeit werden erhalten, wenn zur Herstellung der Bindemittel Silicium enthaltende Vinylmonomere, z. B. Vinyltrimethylsilan, mitverwendet werden. Die Transparenz dieser Bindemittel ist im interessierenden Bereich im allgemeinen höher, so daß eine verbesserte Strukturierung möglich ist.

Mit gleichem Erfolg lassen sich auch Homo- oder Copolymere des Maleimids verwenden. Auch diese Bindemittel zeigen hohe Transparenz im beschriebenen Wellenlängenbereich. Als Comonomere werden auch hier bevorzugt Styrol, substituierte Styrole, Vinylether, Vinylester, Vinylsilylverbindungen oder (Meth)acrylsäureester eingesetzt.

Schließlich sind darüber hinaus auch Copolymere des Styrols mit Comonomeren verwendbar, die in wäßrig alkalischen Lösungen eine Löslichkeitserhöhung bewirken. Hierzu zählen beispielsweise Maleinsäureanhydrid und Maleinsäurehalbester.

Die genannten Bindemittel können gemischt werden, sofern sich die optische Qualität des strahlungsempfindlichen Gemisches dadurch nicht verschlechtert. Bindemittelgemische sind jedoch nicht bevorzugt.

Die Glasübergangstemperatur des Bindemittels bzw. der Bindemittelgemische liegt vorteilhaft bei mindestens 120 °C.

Der Anteil des Bindemittels beträgt im allgemeinen 40 bis 95 Gew.-%, insbesondere 50 bis 90 Gew.-%, bezogen auf das Gesamtgewicht der festen Anteile des strahlungsempfindlichen Gemisches.

Ferner können den erfindungsgemäßen strahlungsempfindlichen Gemischen gegebenenfalls Farbstoffe, Pigmente, Weichmacher, Netzmittel und Verlaufmittel, aber auch Polyglykole, Celluloseether, z. B. Ethylcellulose, zur Erfüllung spezieller Erfordernisse, wie Flexibilität, Haftung und Glanz, zugesetzt werden.

Soll ein Substrat beschichtet werden, so wird das erfindungsgemäße strahlungsempfindliche Gemisch zweckmäßig in einem Lösemittel oder in einer Kombination von Lösemitteln gelöst. Letztendlich hängt die Wahl des Lösemittels bzw. Lösemittelgemisches ab von dem angewandten Beschichtungsverfahren, der gewünschten Schichtstärke und den Trocknungsbedingungen. Ebenso müssen die Lösemittel unter den angewendeten Bedingungen gegenüber den übrigen Schichtbestandteilen chemisch inert sein. Besonders geeignete Lösemittel sowie die angewandten Beschichtungsverfahren sind in der nicht vorveröffentlichten deutschen Patentanmeldung P 41 12 971.7 ausführlich beschrieben und gelten hier analog.

Die mit den genannten Lösemitteln hergestellte Lösung hat in der Regel einen Feststoffgehalt von 5 bis 60 Gew.-%, vorzugsweise bis 50 Gew.-%.

Gegenstand der Erfindung ist schließlich auch ein strahlungsempfindliches Aufzeichnungsmaterial, das im wesentlichen aus einem Substrat und einer darauf befindlichen strahlungsempfindlichen Schicht aus dem erfindungsgemäßen strahlungsempfindlichen Gemisch besteht.

Als Substrate kommen alle Materialien in Frage, aus denen Kondensatoren, Halbleiter, mehrlagige gedruckte Schaltungen oder integrierte Schaltkreise bestehen bzw. hergestellt werden können. Gegebenenfalls wird ein Haftvermittler eingesetzt. Die aus der nicht vorveröffentlichten deutschen Patentanmeldung P 41 12 971.7 bekannten Substrate, Haftvermittler und Trägermaterialien zur Herstellung photomechanischer Aufzeichnungsschichten werden auch hier analog angewendet.

Das erfindungsgemäße Aufzeichnungsmaterial wird mit aktinischer Strahlung bildmäßig belichtet. Geeignete Strahlungsquellen werden ebenfalls in der vorgenannten Schrift ausführlich beschrieben und hier entsprechend eingesetzt.

Die Stärke der lichtempfindlichen Schicht hängt vom Verwendungszweck ab. Sie beträgt im allgemeinen zwischen 0,1 und 100 µm, bevorzugt zwischen 0,5 und 10 µm, besonders bevorzugt um 1,0 µm.

Gegenstand der Erfindung ist ferner ein Verfahren zur Herstellung eines strahlungsempfindlichen Aufzeichnungsmaterials. Für das Auftragen des strahlungsempfindlichen Gemisches auf das Substrat gelten die in der P 41 12 971.7 gemachten Ausführungen ebenso wie für die als Entwickler geeigneten wäßrigen Lösungen, das Nachhärten von entwickelten Schichtstrukturen und die Verwendung des erfindungsgemäßen strahlungsempfindlichen Gemischs bei der Herstellung von integrierten Schaltungen oder von einzelnen elektrischen Bausteinen mit lithographischen Prozessen.

Die erfindungsgemäßen strahlungsempfindlichen Gemische weisen besonders bei Bestrahlung mit Licht einer Wellenlänge zwischen 190 bis 300 nm eine hohe Lichtempfindlichkeit auf. Da die Gemische eine ausgesprochen hohe Transparenz im gewünschten Wellenlängenbereich aufweisen, lassen sich feinere Strukturen erzielen als dies mit bekannten Gemischen möglich ist. Die entwickelte Resistschicht dient dabei als Maske für die folgenden Prozeßschritte. Solche Schritte sind z. B. das Ätzen des Schichtträgers, das Implantieren von Ionen in den Schichtträger oder das Abscheiden von Metallen oder anderen Materialien auf dem Schichtträger.

Die nachstehend beschriebenen Beispiele illustrieren die Erfindung, sollen aber keine Einschränkung bewirken. Gt steht im Folgenden für Gewichtsteile, Vt für Volumenteile. Gt verhalten sich zu Vt wie g zu $cm^3$.

Herstellungsbeispiel 1:

1. Stufe: 25,0 Gt 4-Cyanophenol und 31,2 Gt Triethylamin werden in 200 Gt Tetrahydrofuran gelöst und auf 5°C gekühlt. Unter Rühren und Konstanthalten der Temperatur werden 39,8 Gt p-Toluolsulfonsäurechlorid, gelöst in 50 Gt Tetrahydrofuran, zugetropft. Die Mischung wird auf Raumtemperatur gebracht, 2 Stunden nachgerührt und in 1000 Vt destilliertes Wasser eingerührt. Nach dem Ansäuern auf pH 2 bis 3 wird der Niederschlag abgesaugt, mit Wasser neutral gewaschen und getrocknet. Das kristalline Material wird aus Isopropanol umkristallisiert. Man erhält 53 Gt 4-(Toluol-4-sulfonyloxy)-benzonitril (weiße Kristalle mit einem Schmelzpunkt von 85°C).

2. Stufe: 13,6 Gt der vorstehend beschriebenen Verbindung werden unter Feuchtigkeitsausschluß in 43,4 Gt Trichloracetonitril gelöst und mit 2,7 Gt Aluminiumbromid versetzt. In diese Mischung wird bei 24 bis 30°C trockenes Chlorwasserstoffgas bis zur Sättigung eingeleitet. Die sich verfestigende Mischung läßt man 24 Stunden bei Raumtemperatur stehen. Sie wird dann in 400 Gt Methylenchlorid aufgenommen und zweimal mit je 100 Gt Wasser gewaschen. Die organische Phase wird getrocknet, filtriert und eingeengt. Der Rückstand wird aus Ethanol umkristallisiert. Man erhält 23,5 Gt 2,4-Bis-trichlormethyl-6-[4-(toluol-4-sulfonyloxy)-phenyl]-[1,3,5]triazin (weiße Kristalle mit einem Schmelzpunkt von 156°C).

Herstellungsbeispiel 2:

1. Stufe: 25,0 Gt 4-Cyanophenol und 31,2 Gt Triethylamin werden in 200 Gt Tetrahydrofuran gelöst und auf 5°C gekühlt. Unter Rühren und Konstanthalten der Temperatur werden 23,3 Gt Methansulfonsäurechlorid, gelöst in 50 Gt Tetrahydrofuran, zugetropft. Die Mischung wird auf Raumtemperatur gebracht, 2 Stunden nachgerührt und in 1000 Vt destilliertes Wasser eingerührt. Nach dem Ansäuern auf pH 2 bis 3 wird der Niederschlag abgesaugt, mit Wasser neutral gewaschen und getrocknet. Das kristalline Material wird aus Isopropanol umkristallisiert. Man erhält 35 Gt 4-Methansulfonyloxy-benzonitril (weiße Kristalle mit einem Schmelzpunkt von 93°C).

2. Stufe: 9,8 Gt der vorstehend beschriebenen Verbindung werden unter Feuchtigkeitsausschluß in 43,4 Gt Trichloracetonitril gelöst und mit 2,7 Gt Aluminiumbromid versetzt. In diese Mischung wird bei 24 bis 30°C trockenes Chlorwasserstoffgas bis zur Sättigung eingeleitet. Die sich verfestigende Mischung wird 24 Stunden bei Raumtemperatur stehengelassen. Sie wird in 400 Gt Methylenchlorid aufgenommen und zweimal mit je 100 Gt Wasser gewaschen. Die organische Phase wird getrocknet, filtriert und eingeengt. Der Rückstand wird aus Ethanol umkristallisiert. Man erhält 13,8 Gt 2,4-Bis-trichlormethyl-6-(4-methansulfonyloxy-phenyl)-[1,3,5]triazin (weiße Kristalle mit einem Schmelzpunkt von 142°C).

Herstellungsbeispiel 3:

1. Stufe: 11,9 Gt 4-Cyanophenol und 15,0 Gt Triethylamin werden in 120 Gt Tetrahydrofuran gelöst und auf 0°C gekühlt. Dazu werden unter Rühren und Konstanthalten der Temperatur 22,6 Gt Naphthalin-2-sulfonylchlorid, gelöst in 50 Gt Tetrahydrofuran getropft. Die Mischung wird auf Raumtemperatur gebracht, 2 Stunden nachgerührt und in 1000 Vt destilliertes Wasser eingerührt. Nach dem Ansäuern auf pH 2 bis 3 wird der Niederschlag abgesaugt, mit Wasser neutral gewaschen und getrocknet. Das kristalline Material wird aus Isopropanol umkristallisiert. Man erhält 25,3 Gt 4-(Naphthalin-2-sulfonyloxy)-benzonitril (weiße Kristalle mit einem Schmelzpunkt von 112°C).

2. Stufe: 23,2 Gt der vorstehend beschriebenen Verbindung werden unter Feuchtigkeitsausschluß in 65,0 Gt Trichloracetonitril gelöst und mit 4,0 Gt Aluminiumbromid versetzt. In diese Mischung wird bei 24 bis 30°C trockenes Chlorwasserstoffgas bis zur Sättigung eingeleitet. Die sich verfestigende Mischung läßt man 24 Stunden bei Raumtemperatur stehen. Sie wird dann in 400 Gt Methylenchlorid aufgenommen und zweimal mit je 100 Gt Wasser gewaschen. Die organische Phase wird getrocknet, filtriert und eingeengt. Der Rückstand wird aus Ethylacetat umkristallisiert. Man erhält 32,5 Gt 2,4-Bis-trichlormethyl-6-[4-(naphthalin-2-sulfonyloxy)-phenyl]-[1,3,5]triazin (weiße Kristalle mit einem Schmelzpunkt von 175°C).

Herstellungsbeispiel 4:

1. Stufe: 6,0 Gt 4-Cyanophenol und 7,5 Gt Triethylamin werden in 200 Gt Tetrahydrofuran gelöst und auf

5°C gekühlt. Dazu werden unter Rühren und Konstanthalten der Temperatur 10,3 Gt 4-Methoxybenzolsulfonylchlorid, gelöst in 50 Gt Tetrahydrofuran, getropft. Die Mischung wird auf Raumtemperatur gebracht, 2 Stunden nachgerührt und in 1000 Vt destilliertes Wasser eingerührt. Nach dem Ansäuern auf pH 2 bis 3 wird der Niederschlag abgesaugt, mit Wasser neutral gewaschen und getrocknet. Das kristalline Material wird aus Isopropanol umkristallisiert. Man erhält 13 Gt 4-(4-Methoxybenzolsulfonyloxy)-benzonitril (weiße Kristalle mit einem Schmelzpunkt von 128°C).
2. Stufe: 11,6 Gt der vorstehend beschriebenen Verbindung werden unter Feuchtigkeitsausschluß in 35,0 Gt Trichloracetonitril gelöst und mit 2,5 Gt Aluminiumbromid versetzt. In diese Mischung wird bei 24 bis 30°C trockenes Chlorwasserstoffgas bis zur Sättigung eingeleitet. Die sich verfestigende Mischung läßt man 24 Stunden bei Raumtemperatur stehen. Sie wird in 300 Gt Methylenchlorid aufgenommen und zweimal mit je 100 Gt Wasser gewaschen. Die organische Phase wird getrocknet, filtriert und eingeengt. Der Rückstand wird aus Ethylacetat umkristallisiert. Man erhält 16,8 Gt 2,4-Bis-trichlormethyl-6-[4-(4-methoxybenzolsulfonyloxy)-phenyl]-[1,3,5]triazin (weiße Kristalle mit einem Schmelzpunkt von 168°C).

Herstellungsbeispiel 5:

1. Stufe: 4,0 Gt 3-Cyanophenol und 5,1 Gt Triethylamin werden in 100 Gt Tetrahydrofuran gelöst und auf 5°C gekühlt. Dazu werden unter Rühren und Konstanthalten der Temperatur 10,0 Gt 2,4,6-Triisopropylbenzolsulfonylchlorid, gelöst in 50 Gt Tetrahydrofuran, getropft. Die Mischung wird auf Raumtemperatur gebracht, 2 Stunden nachgerührt und in 1000 Vt destilliertes Wasser eingerührt. Nach dem Ansäuern auf pH 2 bis 3 wird der Niederschlag abgesaugt, mit Wasser neutral gewaschen und getrocknet. Das kristalline Material wird aus Isopropanol umkristallisiert. Man erhält 12 Gt 3-(2,4,6-Triisopropylbenzolsulfonyloxy)-benzonitril (weiße Kristalle mit einem Schmelzpunkt von 70°C).
2. Stufe: 11,5 Gt der vorstehend beschriebenen Verbindung werden unter Feuchtigkeitsausschluß in 26,0 Gt Trichloracetonitril gelöst und mit 2,0 Gt Aluminiumbromid versetzt. In diese Mischung wird bei 24 bis 30°C trockenes Chlorwasserstoffgas bis zur Sättigung eingeleitet. Die sich verfestigende Mischung läßt man 24 Stunden bei Raumtemperatur stehen. Sie wird in 200 Gt Methylenchlorid aufgenommen und zweimal mit je 100 Gt Wasser gewaschen. Die organische Phase wird getrocknet, filtriert und eingeengt. Der Rückstand wird aus Ethylacetat umkristallisiert. Man erhält 10,5 Gt 2,4-Bis-trichlormethyl-6-[3-(2,4,6-triisopropyl-benzolsulfonyloxy)-phenyl]-[1,3,5]triazin (weiße Kristalle mit einem Schmelzpunkt von 156°C).

Herstellungsbeispiel 6:

1. Stufe: 11,9 Gt 2-Cyanophenol und 15,2 Gt Triethylamin werden in 100 Gt Tetrahydrofuran gelöst und auf 5°C gekühlt. Dazu werden unter Rühren und Konstanthalten der Temperatur 11,4 Gt Methansulfonsäurechlorid, gelöst in 50 Gt Tetrahydrofuran, getropft. Die Mischung wird auf Raumtemperatur gebracht, 2 Stunden nachgerührt und in 1000 Vt destilliertes Wasser eingerührt. Nach dem Ansäuern auf pH 2 bis 3 wird der Niederschlag abgesaugt, mit Wasser neutral gewaschen und getrocknet. Das kristalline Material wird aus Isopropanol umkristallisiert. Man erhält 13,5 Gt 2-Methansulfonyloxy-benzonitril (weiße Kristalle mit einem Schmelzpunkt von 58°C).
2. Stufe: 12,8 Gt der vorstehend beschriebenen Verbindung werden unter Feuchtigkeitsausschluß in 56,6 Gt Trichloracetonitril gelöst und mit 2,0 Gt Aluminiumbromid versetzt. In diese Mischung wird bei 24 bis 30°C trockenes Chlorwasserstoffgas bis zur Sättigung eingeleitet. Die sich verfestigende Mischung läßt man 24 Stunden bei Raumtemperatur stehen. Sie wird in 200 Gt Methylenchlorid aufgenommen und zweimal mit je 100 Gt Wasser gewaschen. Die organische Phase wird getrocknet, filtriert und eingeengt. Der Rückstand wird aus Isopropanol umkristallisiert. Man erhält 15,7 Gt 2,4-Bis-trichlormethyl-6-(2-methansulfonyloxy-phenyl)-[1,3,5]triazin (weiße Kristalle mit einem Schmelzpunkt von 86°C).

Herstellungsbeispiele 7 bis 13

Es werden weitere Verbindungen der allgemeinen Formel I vorgestellt, die analog zu den bisher beschriebenen Beispielen hergestellt wurden. Wegen der Vielzahl der Verbindungen werden einige ausgewählte Vertreter in der folgenden Tabelle hinsichtlich der in der allgemeinen Formel I beschriebenen Variationsmöglichkeiten charakterisiert. Als Analysenwert ist die quantitative Bestimmung des Schwefel- und des Chlorgehalts hinreichend aussagefähig.

Tabelle

| Nr. | Position *) | R | Schmp. [°C] | Elementaranalyse % Cl ber. | gef. | % S ber. | gef. |
|---|---|---|---|---|---|---|---|
| 7 | para | $-C_6H_5$ | 140 | 38,8 | 38,2 | 5,8 | 5,3 |
| 8 | para | $-2,4,5-(Cl)_3-C_6H_2$ | 190 | 49,0 | 49,0 | 4,9 | 5,2 |
| 9 | para | $-4-Cl-C_6H_4$ | 150 | 42,6 | 43,1 | 5,5 | 5,4 |
| 10 | para | $-4-Br-C_6H_4$ | 148 | 33,9 | 34,2 | 5,1 | 5,2 |
| 11 | meta | $-4-CH_3-C_6H_4$ | 162 | 37,8 | 38,1 | 5,7 | 6,0 |
| 12 | meta | $-CH_3$ | 120 | 43,8 | 48,5 | 6,6 | 5,9 |
| 13 | ortho | $-4-CH_3-C_6H_4$ | 86 | 37,8 | 39,4 | 5,7 | 6,1 |

*) Bezogen auf die Stellung der 2,4-Bis-trichlormethan-[1,3,5]triazin-6-yl-Gruppe am Benzolring.

Auf analoge Weise lassen sich auch andere der weiter oben beschriebenen, mit Sulfonsäuren der allgemeinen Formel $R-SO_3H$ bzw. $R'(-SO_3H)_2$ veresterten 2,4-Bis-trichlormethyl-6-(mono- oder dihydroxyphenyl)-[1,3,5]triazine herstellen.

Die Charakterisierung der mit Sulfonsäure(n) der Formel $R-SO_3H$ bzw. $R'(-SO_3H)_2$ veresterten 2,4-Bis-trichlormethyl-6-(mono- oder dihydroxyphenyl)-[1,3,5]triazine erfolgte durch $^1$H-Hochfeld-Kernresonanzspektren sowie durch Elementaranalysen und durch IR-Spektroskopie zum Beweis der Abwesenheit freier Hydroxygruppen im Produkt.

Anwendungsbeispiele

Die Beispiele 1 bis 7 belegen die Eignung des erfindungsgemäßen Gemisches für Aufzeichnungsmaterialien in der Mikrolithographie. Anhand der Vergleichsbeispiele 8 und 9 wird die Überlegenheit der erfindungsgemäßen Gemische sche gegenüber dem Stand der Technik belegt. Die Beispiele 10 und 11 dokumentieren die Anwendbarkeit des Gemisches in gedruckten Schaltungen und Flachdruckplatten.

Die Beschichtungslösungen wurden durch Filter mit einem Porendurchmesser von 0,2 µm filtriert und auf einen, mit einem Haftvermittler (Hexamethyldisilazan) vorbehandelten, Wafer aufgeschleudert. Die Schleuderdrehzahl wurde dabei so gewählt, daß man nach 1 min Trocknen bei 100 °C auf der hot plate Schichtdicken um 1,05 µm erhielt.

Sofern bei den einzelnen Beispielen nichts anderes angegeben ist, wurde das Aufzeichnungsmaterial bildmäßig unter einer Vorlage mit der Strahlung eines KrF-Excimer-Lasers (248 nm) oder einer Xenon-Quecksilberdampflampe (260 nm, mit Interferenzfilter) belichtet und anschließend 1 min bei 100 °C einem post exposure bake auf einer hot plate unterzogen.

Entwickelt wurde das Aufzeichnungsmaterial mit einer 0,27 n wäßrigen Tetramethylammoniumhydroxid-Lösung.

Beispiel 1:

Es wurde ein lichtempfindliches Aufzeichnungsmaterial hergestellt mit Hilfe einer Beschichtungslösung, bestehend aus

| | |
|---|---|
| 7,5 Gt | eines Copolymeren aus Styrol und Maleimid (Molverhältnis 50:50) mit einem Erweichungsbereich von 165 bis 180 °C, |
| 2,0 Gt | Hexa-N-acetoxymethyl-melamin und 0,075 Gt 2,4-Bis-trichlormethyl-6-[4-(4-methoxybenzolsulfonyloxy)-phenyl]-[1,3,5]triazin gemäß dem Herstellungsbeispiel 4, |

| | |
|---|---|
| | in |
| 42 Gt | Propylenglykol-monomethylether-acetat. |
| Softbake: | 1 min, 110 °C, hot plate Belichtung: 3,8 mJ/cm² (Xenon-Quecksilberdampflampe) |
| Post exposure bake: | 2 min, 120 °C, hot plate |
| Entwicklung: | 120 s (0,02 n wäßrige Tetramethylammoniumhydroxid-Lösung) |

Beispiel 2:

Es wurde ein lichtempfindliches Aufzeichnungsmaterial hergestellt mit Hilfe einer Beschichtungslösung, bestehend aus

| | |
|---|---|
| 7,5 Gt | des in Beispiel 1 genannten Copolymeren, |
| 2,5 Gt | 4,4'-Bis-methoxymethyl-diphenylether und |
| 0,15 Gt | 2,4-Bis-trichlormethyl-6-[3-(toluol-4-sulfonyloxy)-phenyl]-[1,3,5]triazin gemäß dem Herstellungsbeispiel 11, in |
| 42 Gt | Propylenglykol-monomethylether-acetat. |
| Softbake: | 1 min, 110 °C, hot plate |
| Belichtung: | 2,8 mJ/cm² (Xenon-Quecksilberdampflampe) |
| Post exposure bake: | 2 min, 115 °C, hot plate |
| Entwicklung: | 90 s (0,02 n wäßrige Tetramethylammoniumhydroxid-Lösung) |

Beispiel 3

Es wurde ein lichtempfindliches Aufzeichnungsmaterial hergestellt mit Hilfe einer Beschichtungslösung, bestehend aus

| | |
|---|---|
| 7,5 Gt | eines Homopolymeren aus 3-Methyl-4-hydroxystyrol mit einem Erweichungsbereich von ≧ 150 °C (GPC), |
| 2,5 Gt | eines Kresol-Formaldehyd-Resols ((R)Bakelite R5363) und |
| 0,14 Gt | 2,4-Bis-trichlormethyl-6-[4-(toluol-4-sulfonyloxy)-phenyl]-[1,3,5]triazin gemäß dem Herstellungsbeispiel 1, in |
| 42 Gt | Propylenglykol-monomethylether-acetat. |
| Belichtung: | 2,9 mJ/cm² (Xenon-Quecksilberdampflampe) |
| Entwicklung: | 90 s |

Beispiel 4:

Es wurde ein lichtempfindliches Aufzeichnungsmaterial hergestellt mit Hilfe einer Beschichtungslösung gemäß Beispiel 3, mit der Abweichung, daß anstelle von 2,5 Gt eines Kresol-Formaldehyd-Resols ((R)Bakelite R5363) 2,0 Gt Hexa-N-methoxymethyl-melamin verwendet wurden.

| | |
|---|---|
| Belichtung: | 2,7 mJ/cm² (KrF-Excimer-Laser) |
| Entwicklung: | 105 s |

Beispiel 5:

Es wurde ein lichtempfindliches Aufzeichnungsmaterial hergestellt mit Hilfe einer Beschichtungslösung, bestehend aus

| | |
|---|---|
| 7,5 Gt | eines Copolymeren aus Styrol/4-Hydroxystyrol (Molverhältnis 20:80) mit einem mittleren Molekulargewicht um 30.000 (GPC), |
| 2,5 Gt | Hexa-N-butoxymethyl-melamin und |
| 0,1 Gt | 2,4-Bis-trichlormethyl-6-[4-(2,4,5-trichlorbenzol-sulfonyloxy)-phenyl]-[1,3,5]triazin gemäß dem Herstellungsbeispiel 8, in |
| 42 Gt | Propylenglykol-monomethylether-acetat. |
| Softbake: | 1 min, 90 °C, hot plate |
| Belichtung: | 3,4 mJ/cm² (KrF-Excimer-Laser) |
| Post exposure bake: | 120 s, 100 °C, hot plate |
| Entwicklung: | 120 s (0,135 n wäßrige Tetramethylammoniumhydroxid-Lösung) |

Beispiel 6:

Es wurde ein lichtempfindliches Aufzeichnungsmaterial hergestellt mit Hilfe einer Beschichtungslösung, bestehend aus

| | |
|---|---|
| 8,0 Gt | eines Copolymeren aus 3-Methyl-4-hydroxystyrol/p-Hydroxystyrol (Molverhältnis 75:25) mit einem Erweichungsbereich von > 150 °C und einem mittleren Molekulargewicht von 28.000 (GPC), |
| 2,0 Gt | Hexa-N-methoxymethyl-melamin und |
| 0,05 Gt | 2,4-Bis-trichlormethyl-6-(4-benzolsulfonyloxyphenyl)-[1,3,5]triazin gemäß dem Herstellungsbeispiel 7, in |
| 42 Gt | Propylenglykol-monomethylether-acetat. |
| Belichtung: | 4,1 mJ/cm² (Xenon-Quecksilberdampflampe) |
| Entwicklung: | 75 s |

Beispiel 7:

Es wurde ein lichtempfindliches Aufzeichnungsmaterial hergestellt mit Hilfe einer Beschichtungslösung, bestehend aus

| | |
|---|---|
| 7,5 Gt | des in Beispiel 1 genannten Copolymeren, |
| 2,5 Gt | 4,4'-Bis-methoxymethyldiphenylsulfon und |
| 0,1 Gt | 2,4-Bis-trichlormethyl-6-(4-methansulfonyloxyphenyl)-[1,3,5]triazin gemäß dem Herstellungsbeispiel 2, in |
| 42 Gt | Propylenglykol-monomethylether-acetat. |
| Belichtung: | 3,0 mJ/cm² (Xenon-Quecksilberdampflampe) |
| Entwicklung: | 75 s (0,02 n wäßrige Tetramethylammoniumhydroxid-Lösung) |

Bewertung der entwickelten Aufzeichnungsmaterialien

Die gemäß den Beispielen 1 bis 7 erhaltenen Resiststrukturen stellten ein fehlerfreies, negatives Abbild der Maske mit steilen Resistflanken dar, wobei auch Strukturen im Sub-Halbmikrometer-Bereich detailgetreu wiedergegeben waren. Eine rasterelektronenmikroskopische Untersuchung zeigte, daß die Resistflanken senkrecht zur Substratoberfläche ausgerichtet waren.

Die Schichtabträge in den belichteten Resistbereichen lagen in allen Fällen bei 15 nm/min und darunter.

Beispiele 8 und 9 (Vergleichsbeispiele)

Die Resistformulierung des Beispiels 3 wurde derart abgeändert, daß die dort verwendete säurebildende Verbindung durch die gleiche Menge an 2,4-Bis-trichlormethyl-6-(4-methoxy-naphthalin-1-yl)-[1,3,5]triazin (Photoinitiator Nr. 3 in Tabelle 2 der DE-A 27 18 259) oder 4,6,4',6'-Tetrakis-trichlormethyl-2,2-p-phenylen-bis-[1,3,5]triazin, hergestellt gemäß dem in der DE-A angegebenen Verfahren, ersetzt wurde.

Der Versuch, eine Beschichtungslösung entsprechend der in Beispiel 7 angegebenen Zusammensetzung herzustellen, mißlang, da die säurebildende Verbindung in der in Beispiel 7 angegebenen Konzentration keine ausreichende Löslichkeit im Resistlösemittel aufweist. Erst eine deutlich verringerte Konzentration des photoaktiven Säurebildners führt zu einer homogenen Beschichtungslösung. Diese Resistformulierungen zeigen jedoch eine wesentlich verringerte Empfindlichkeit (> 8 mJ/cm²) gegenüber den erfindungsgemäßen Gemischen.

Verschiedene Versuche der Bestrahlung eines - mit einer Beschichtungslösung nach Beispiel 8 - beschichteten Wafers wiesen deutliche Unterschiede in den Lichtempfindlichkeiten auf. Analytische Untersuchungen der Beschichtungslösung mit HPLC (High-pressure-liquid-chromatography) vor und nach dem Trocknungsprozeß (Softbake) - ohne nachfolgenden Belichtungsschritt - zeigten, daß beträchtliche Mengen der säurebildenden Verbindung aus der Schicht ausdampften. Obwohl die Größenordnung der Sensitivitätsschwankungen weniger stark ausgeprägt war als in vergleichbaren Positivsystemen (siehe die gleichzeitig eingereichte deutsche Patentanmeldung P 41 20 173.6), macht dieses Verhalten die für eine praxisgerechte Prozeßführung erforderliche Kontrolle der Lichtempfindlichkeit nahezu unmöglich. Bei vergleichbaren Untersuchungen der erfindungsgemäßen strahlungsempfindlichen Gemische wurde ein solches Verhalten in keinem Fall beobachtet.

Beispiel 10:

Für die Herstellung einer Offsetdruckplatte wurde eine mechanisch aufgerauhte und vorbehandelte Aluminiumfolie mit einer Beschichtungslösung nachstehender Zusammensetzung schleuderbeschichtet:

| | |
|---|---|
| 7,5 Gt | eines Kresol-Formaldehyd-Novolaks mit einem Erweichungsbereich von 105 bis 120 °C, |
| 2,5 Gt | eines Kresol/Formaldehyd Resols ((R)Bakelite R 5363), |
| 0,2 Gt | 2,4-Bis-trichlormethyl-6-[4-(toluol-4-sulfonyloxy)-phenyl]-[1,3,5]triazin gemäß dem Herstellungsbeispiel 1, und |
| 0,05 Gt | Kristallviolettbase in |
| 90 Gt | Propylenglykol-monomethylether-acetat. |

Nach dem Trocknen der Schicht (Schichtgewicht ca. 2,5 $g/m^2$) wurde unter einer negativen Testvorlage 45 s belichtet und die Platte nach 10 minütiger Lagerung für 2 min in einem Umluftofen mit einer Temperatur von 140 °C erwärmt. Es wurde mit einem Entwickler folgender Zusammensetzung entwickelt:

| | |
|---|---|
| 0,5 Gt | Natriumhydroxid, |
| 0,8 Gt | Natriummetasilikat x 9 $H_2O$ und |
| 1,0 Gt | 2-n-Butoxy-ethanol in |
| 97,7 Gt | vollentsalztem Wasser. |

Bei der Entwicklung wurde ein detailgetreues negatives Abbild der Vorlage sichtbar. Nach Abspülen mit Wasser wurde die Platte durch Überwischen mit 1%iger Phosphorsäure druckfertig gemacht. Von dieser Druckplatte wurden 135.000 einwandfreie Drucke erhalten.

Beispiel 11:

Für die Herstellung eines Ätz- und Galvanonegativtrockenresists wurde eine Lösung nachstehender Zusammensetzung bereitet:

| | |
|---|---|
| 12,5 Gt | des in Beispiel 10 beschriebenen Novolaks, |
| 10,0 Gt | Hexa-N-methoxymethyl-melamin, |
| 0,12 Gt | 2,4-Bis-trichlormethyl-6-[3-(2,4,6-triisopropyl-benzolsulfonyloxy)-phenyl]-[1,3,5]-triazin gemäß dem Herstellungsbeispiel 5, und |
| 0,1 Gt | Kristallviolett in |
| 30 Gt | Butanon. |

Eine für diesen Zweck übliche Polyethylenterephthalatfolie von 25 µm Dicke wurde mit dieser Lösung beschichtet, so daß sich eine Trockenschichtdicke von 18 µm ergab. Die Oberfläche des trockenen Resistfilms wurde mit einer weiteren Polyethylenterephthalatfolie kaschiert. Der Trockenfilm wurde nach Abziehen der Deckfolie unter Druck und Wärme auf ein Messingblech auflaminiert. Nach Abkühlen und Abziehen der Trägerfolie wurde das Blech durch eine Vorlage hindurch belichtet, wobei ein guter Bildkontrast sichtbar wurde. Das Material wurde 10 min gelagert und dann für 4 min auf 95 °C erwärmt. Die unbelichteten Stellen wurden mit einem Entwickler der in Beispiel 10 angegebenen Zusammensetzung sprühentwickelt. Das Blech wurde anschließend mit handelsüblicher Eisen-III-chlorid-Lösung bis hin zu den glatten Flanken durchgeätzt. Es ist möglich, die erhaltenen Formteile vor dem Trennen noch weiter zu bearbeiten.

## Patentansprüche

1. Strahlungsempfindliche, mit Sulfonsäuren der Formel $R-SO_3H$ bzw. $R'(-SO_3H)_2$ veresterte 2,4-Bis-trichlormethyl-6-(mono- oder dihydroxyphenyl)-[1,3,5]-triazine der allgemeinen Formeln I und/oder II

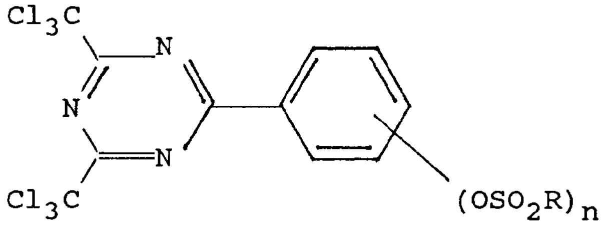

(I)

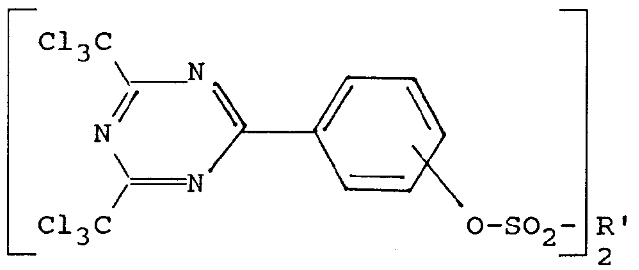

(II)

wobei R einen ggf. weiter substituierten $(C_1\text{-}C_{10})$Alkyl-, $(C_5\text{-}C_{10})$Cycloalkyl-, $(C_6\text{-}C_{10})$Aryl-, $(C_6\text{-}C_{10})$Aryl-$(C_1\text{-}C_{10})$alkyl-, $(C_3\text{-}C_9)$Heteroarylrest, 1-Anthracenyl oder 2-Anthrachinonyl, R′ einen ggf. substituierten $(C_1\text{-}C_{10})$Alkylen-, $(C_6\text{-}C_{10})$Arylen- oder $(C_3\text{-}C_9)$Heteroarylenrest bezeichnet und n 1 oder 2 sein kann.

2. Sulfonsäureester gemäß Anspruch 1, dadurch gekennzeichnet, daß die 2,4-Bis-trichlormethyl-6-(dihydroxyphenyl)-[1,3,5]triazine der allgemeinen Formel I vollständig mit der Sulfonsäure der Formel $R\text{-}SO_3H$ verestert sind.

3. Sulfonsäureester gemäß einem der Ansprüche 1 oder 2, dadurch gekennzeichnet, daß die Reste R oder R′ mit mindestens einem Substituenten ausgewählt aus der Gruppe bestehend aus $(C_1\text{-}C_8)$Alkyl, $(C_1\text{-}C_8)$ Alkoxy, $(C_1\text{-}C_8)$Alkanoyl, $(C_1\text{-}C_8)$Alkanoyloxy, $(C_6\text{-}C_{10})$Aryl, Cyano oder Halogen substituiert sind.

4. Negativ arbeitendes strahlungsempfindliches Gemisch, das
a) eine Verbindung, die unter Einwirkung von aktinischer Strahlung starke Säure bildet,
b) eine Verbindung mit mindestens zwei durch Säure vernetzbaren Gruppen und
c) ein in Wasser unlösliches, in wäßrig-alkalischen Lösungen lösliches oder zumindest quellbares polymeres Bindemittel,
enthält, dadurch gekennzeichnet, daß die Verbindung a) ein Sulfonsäureester gemäß einem oder mehreren der Ansprüche 1 bis 3 ist.

5. Negativ arbeitendes strahlungsempfindliches Gemisch gemäß Anspruch 4, dadurch gekennzeichnet, daß die Verbindung a) einen Anteil von 0,25 bis 15 Gew.-%, bevorzugt 0,5 bis 5 Gew.-%, am Gesamtgewicht der Feststoffe des Gemisches hat.

6. Negativ arbeitendes strahlungsempfindliches Gemisch gemäß Anspruch 4 oder 5, dadurch gekennzeichnet, daß die Verbindung b) ein Resol ist.

7. Negativ arbeitendes strahlungsempfindliches Gemisch gemäß einem oder mehreren der Ansprüche 4 bis 6, dadurch gekennzeichnet, das die Verbindung b) ein mit Alkoxymethyl- oder Oxiranylmethyl-Gruppen substituierter Aromat ist.

8. Negativ arbeitendes strahlungsempfindliches Gemisch gemäß einem oder mehreren der Ansprüche 4 bis 7, dadurch gekennzeichnet, das die Verbindung b) ein Melamin/- oder Harnstoff/Formaldehyd-Kondensat ist.

9. Negativ arbeitendes strahlungsempfindliches Gemisch gemäß einem oder mehreren der Ansprüche 4 bis 8, dadurch gekennzeichnet, daß die Verbindung mit mindestens zwei durch Säure vernetzbaren Gruppen b) einen Anteil von 1 bis 50 Gew.-%, bevorzugt 5 bis 25 Gew.-%, am Gesamtgewicht der Feststoffe der strahlungsempfindlichen Schicht hat.

10. Negativ arbeitendes strahlungsempfindliches Gemisch gemäß einem oder mehreren der Ansprüche 4 bis 9, dadurch gekennzeichnet, daß das Bindemittel c) phenolische Hydroxygruppen enthält.

11. Negativ arbeitendes strahlungsempfindliches Gemisch gemäß einem oder mehreren der Ansprüche 4 bis 10, dadurch gekennzeichnet, daß das Bindemittel c) für Strahlung der Wellenlänge 248 nm eine Extinktion von $< 0{,}5\ \mu m^{-1}$, bevorzugt $< 0{,}3\ \mu m^{-1}$, aufweist.

12. Negativ arbeitendes strahlungsempfindliches Gemisch gemäß einem oder mehreren der Ansprüche 4 bis

11, dadurch gekennzeichnet, daß das Bindemittel c) einen Anteil von 40 bis 95 Gew.-%, bevorzugt 50 bis 90 Gew.-%, am Gesamtgewicht der Feststoffe des Gemisches hat.

13. Negativ arbeitendes strahlungsempfindliches Aufzeichungsmaterial, im wesentlichen bestehend aus einem Träger und einer strahlungsempfindlichen Schicht, dadurch gekennzeichnet, daß die Schicht ein strahlungshärtbares Gemisch gemäß einem oder mehreren der Ansprüche 4 bis 12 enthält.

## Claims

1. A radiation-sensitive ester of a sulfonic acid of the formula $R\text{-}SO_3H$ or $R'(\text{-}SO_3H)_2$ with 2,4-bis-trichloromethyl-6-(mono- or dihydroxyphenyl)-1,3,5-triazines, said ester having the formulae I and/or II

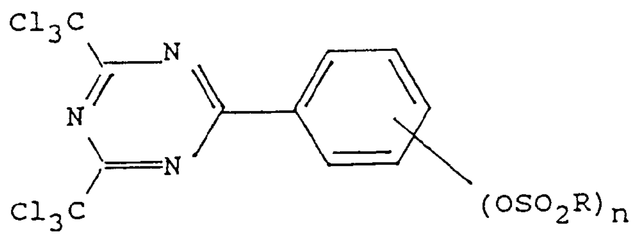

(I)

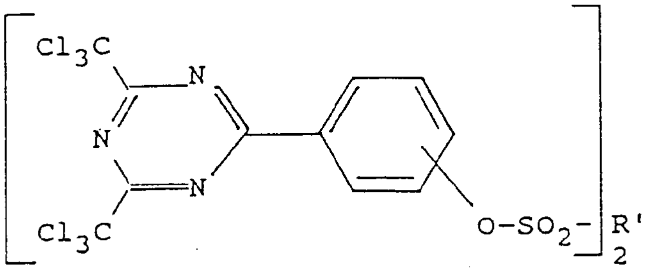

(II)

where R is an optionally further substituted $(C_1\text{-}C_{10})$alkyl, $(C_5\text{-}C_{10})$cycloalkyl, $(C_6\text{-}C_{10})$aryl, $(C_6\text{-}C_{10})$aryl-$(C_1\text{-}C_{10})$alkyl, $(C_3\text{-}C_9)$heteroaryl radical, 1-anthracenyl or 2-anthraquinonyl,
R′ is an optionally substituted $(C_1\text{-}C_{10})$alkylene, $(C_6\text{-}C_{10})$arylene or $(C_3\text{-}C_9)$heteroarylene radical, and n may be 1 or 2.

2. A sulfonic acid ester as claimed in claim 1, wherein the 2,4-bistrichloromethyl-6-dihydroxyphenyl-1,3,5-triazine of the formula I is completely esterified with the sulfonic acid of the formula $R\text{-}SO_3H$.

3. A sulfonic acid ester as claimed in either of claims 1 or 2, wherein the radical R or R′ is substituted with at least one substituent selected from the group comprising $(C_1\text{-}C_8)$alkyl, $(C_1\text{-}C_8)$alkoxy, $(C_1\text{-}C_8)$alkanoyl, $(C_1\text{-}C_8)$alkanoyloxy, $(C_6\text{-}C_{10})$aryl, cyano or halogen.

4. A negative-working radiation-sensitive mixture which contains
   a) a compound which forms strong acid on exposure to actinic radiation,
   b) a compound containing at least two acid-crosslinkable groups, and
   c) a water-insoluble polymeric binder which is soluble or at least swellable in aqueous alkaline solutions,
   wherein the compound a) is a sulfonic acid ester as claimed in one or more of claims 1 to 3.

5. A negative-working radiation-sensitive mixture as claimed in claim 4, wherein the proportion of the compound a) is 0.25 to 15% by weight, preferably 0.5 to 5% by weight, of the total weight of solids in the mixture.

6. A negative-working radiation-sensitive mixture as claimed in claim 4 or 5, wherein the compound b) is a resol.

7. A negative-working radiation-sensitive mixture as claimed in one or more of claims 4 to 6, wherein the

compound b) is an aromatic compound substituted with alkoxymethyl or oxiranylmethyl groups.

8. A negative-working radiation-sensitive mixture as claimed in one or more of claims 4 to 7, wherein the compound b) is a melamine/formaldehyde or urea/formaldehyde condensate.

9. A negative-working radiation-sensitive mixture as claimed in one or more of claims 4 to 8, wherein the proportion of the compound containing at least two acid-crosslinkable groups b) is 1 to 50% by weight, preferably 5 to 25% by weight, of the total weight of solids in the radiation-sensitive layer.

10. A negative-working radiation-sensitive mixture as claimed in one or more of claims 4 to 9, wherein the binder c) contains phenolic hydroxyl groups.

11. A negative-working radiation-sensitive mixture as claimed in one or more of claims 4 to 10, wherein the binder c) has an absorbance of < 0.5 $\mu m^{-1}$, preferably < 0.3 $\mu m^{-1}$, for radiation of the wavelength of 248 nm.

12. A negative-working radiation-sensitive mixture as claimed in one or more of claims 4 to 11, wherein the proportion of the binder c) is from 40 to 95% by weight, preferably 50 to 90% by weight, of the total weight of solids in the mixture.

13. A negative-working radiation-sensitive recording material essentially composed of a base and a radiation-sensitive layer, wherein the layer contains a radiation-curable mixture as claimed in one or more of claims 4 to 12.

## Revendications

1. Composé sensible aux radiations, de type 2,4-bis-trichlorométhyl-6-(mono- ou dihydroxyphényl)-[1,3,5]-triazine estérifiée avec un acide sulfonique de formule R-$SO_3H$, respectivement R'(-$SO_3H)_2$, correspondant à la formule générale I et/ou II

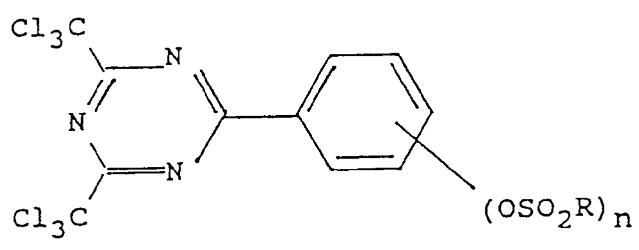

(I)

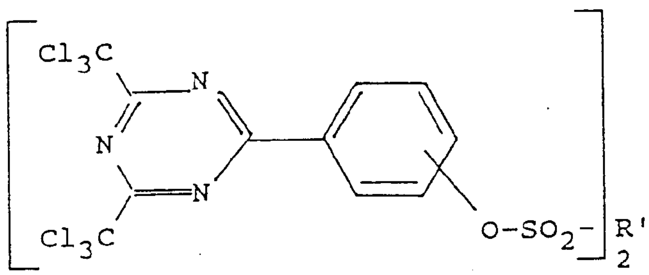

(II)

dans lesquelles R représente un groupe alkyle en $C_1$-$C_{10}$, cycloalkyle en $C_5$-$C_{10}$, aryle en $C_6$-$C_{10}$, (aryle en $C_6$-$C_{10}$)-(alkyle en $C_1$-$C_{10}$), hétéroaryle en $C_3$-$C_9$, 1-anthracényle ou 2-anthraquinonyle, ces groupes étant éventuellement substitués, R' représente un groupe alkylene en $C_1$-$C_{10}$, arylene en $C_6$-$C_{10}$ ou hétéroarylene en $C_3$-$C_9$, ces groupes étant éventuellement substitués, et n peut valoir 1 ou 2.

2. Ester d'acide sulfonique selon la revendication 1, caractérisé en ce que la 2,4-bis-trichlorométhyl-6-(dihydroxyphényle)-[1,3,5]triazine de formule générale I est totalement estérifiée avec l'acide sulfonque de formule $R-SO_3H$.

3. Ester d'acide sulfonique selon l'une des revendications 1 et 2, caractérisé en ce que les groupes R ou R′ sont substitués au moins par un substituant choisi parmi un atome d'halogène, ou les groupes alkyle en $C_1$-$C_8$, alcoxy en $C_1$-$C_8$, alcanoyle en $C_1$-$C_8$, alcanoyloxy en $C_1$-$C_8$, aryle en $C_6$-$C_{10}$ et cyano.

4. Composition sensible aux radiations travaillant en négatif, qui comprend :
   a) un composé qui forme un acide fort sous l'action de radiation actinique,
   b) un composé portant au moins deux groupes réticulables par un acide et
   c) un liant polymère insoluble dans l'eau, soluble ou au moins gonflant dans des solutions aqueuses alcalines, caractérisée en ce que le composé a) est un ester d'acide sulfonique selon l'une ou plusieurs des revendications 1 à 3.

5. Composition sensible aux radiations travaillant en négatif selon la revendication 4, caractérisée en ce que la teneur en composé a) est de 0,25 à 15% en poids, de préférence 0,5 à 5% en poids, par rapport au poids total de solides dans la composition.

6. Composition sensible aux radiations travaillant en négatif selon la revendication 4 ou 5, caractérisée en ce que le composé b) est un résol.

7. Composition sensible aux radiations travaillant en négatif selon l'une ou plusieurs des revendications 4 à 6, caractérisée en ce que le composé b) est un composé aromatique substitué par des groupes alcoxyméthyle ou oxiranylméthyle.

8. Composition sensible aux radiations travaillant en négatif selon l'une ou plusieurs des revendications 4 à 7, caractérisée en ce que le composé b) est un produit de condensation mélamine/formaldéhyde ou urée/formaldéhyde.

9. Composition sensible aux radiations travaillant en négatif selon l'une ou plusieurs des revendications 4 à 8, caractérisée en ce que la teneur en composé b) portant au moins deux groupes réticulables par un acide est de 1 à 50% en poids, de préférence 5 à 25% en poids, par rapport au poids total des solides dans la composition sensible aux radiations.

10. Composition sensible aux radiations travaillant en négatif selon l'une ou plusieurs des revendications 4 à 9, caractérisée en ce que le liant c) contient des groupes hydroxyle phénoliques.

11. Composition sensible aux radiations travaillant en négatif selon l'une ou plusieurs des revendications 4 à 10, caractérisée en ce que le liant c) présente un extinction inférieure à 0,5 $\mu m^{-1}$, de préférence inférieure à 0,3 $\mu m^{-1}$, pour une radiation de longueur d'onde de 248 nm.

12. Composition sensible aux radiations travaillant en négatif selon l'une ou plusieurs des revendications 4 à 11, caractérisée en ce que la teneur en liant c) est de 40 à 95% en poids, de préférence 50 à 90% en poids, par rapport au poids total des solides dans la composition.

13. Matériau de reproduction sensible aux radiations travaillant en négatif, constitué essentiellement d'un support et d'une couche sensible aux radiations, caractérisé en ce que la couche comprend une composition durcissable sous l'effet de radiations selon l'une ou plusieurs des revendications 4 à 12.